# NEW EUROPEAN PATENT SPECIFICATION

(11) **EP 1 294 420 B2**
(45) Date of publication and mention of the opposition decision: **31.08.2016**
(45) Mention of the grant of the patent: 03.12.2008
(21) Application number: 01948660.4
(22) Date of filing: 23.06.2001
(51) Int. Cl.: A61M 15/00

(54) **PRE-METERED DOSE MAGAZINE FOR BREATH-ACTUATED DRY POWDER INHALER**
VORABGEFÜLLTES DOSISMAGAZIN FÜR EINEN ATMUNGSBETÄTIGTEN TROCKENPULVERINHALATOR
MAGASIN A DOSE D'ADMINISTRATION PRE-CALIBREE POUR INHALATEUR A POUDRE SECHE ACTIONNE PAR LA RESPIRATION

(30) Priority: 23.06.2000 US 213667 P; 23.06.2000 US 213382 P
(43) Date of publication of application: 26.03.2003
(73) Proprietor: Norton Healthcare Limited, Castleford, West Yorkshire WF10 5HX (GB)
(72) Inventor: KEANE, Lawrence, Bishop Strotford Hertfordshire CM23 EP (GB); O'LEARY, David, Essex RM17 6UG (GB)
(74) Representative: Miller, James Lionel Woolverton
(86) International application number: PCT/US2001/020097
(87) International publication number: WO 2002/000280

(56) References cited:
- EP-A- 0 525 720
- WO-A-99/36116
- WO-A1-95/16483
- WO-A1-99/36116
- GB-A- 2 264 237
- US-A- 5 388 572
- US-A- 5 458 135
- US-A- 5 622 166
- US-A- 6 006 747
- US-A- 6 065 472

## Description

### Field of the Invention

The invention relates to a breath-actuated dry powder inhaler for administering dry powdermedicament to a patient. More particularly, the present disclosure relates to a magazine having a plurality of individually separated, pre-metered doses for a breath- actuated dry powder inhaler.

### Background of the Invention

Metered dose medicament inhalers are well known for dispensing medicament to the lungs of a patient. In most cases, the inhalers include a reservoir containing dry powder medicament in bulk form, and means for metering the medicament from the reservoir in discrete amounts for inhalation by a patient.

For example, U.S. Patent No. 5,503,144, which is assigned to the assignee of the present disclosure and incorporated herein by reference, shows a breath-actuated dry- powder inhaler having a medicament reservoir. The reservoir contains dry powder medicament in bulk form, and the inhaler includes a metering chamber for removal of the powdered medicament from the reservoir in discrete amounts. The inhaler also includes an air inlet for entraining the removed powdered medicament through a mouthpiece upon patient inhalation.

While the reservoir and metering chamber of the inhaler shown by U. S. Patent No. 5,503,144 properly function to dispense discrete amounts of powdered medicament to a patient, there is desired an inhaler having pre-metered doses of powdered medicament. Providing the powdered medicament in pre-metered doses will further ensure that the medicament is consistently dispensed to a patient in precise doses.

In particular, a device and method are desired for providing individually sealed, pre-metered doses of dry powder medicament for inhalation by a patient through a dry powder inhaler and, in particular, a breath-actuated, dry powder inhaler.

An improved breath-actuated, dry powder inhaler, which substantially de- agglomerates and micronizes pre-metered doses of medicament is also desired to ensure that particles of the medicament are small enough for adequate penetration of the medicament into a bronchial region of a patient's lungs during inhalation.

Reference is also made to the following documents:
WO 99/36116 discloses a device for inhaling medication, comprising: a first housing part provided with one or more cavities in which a dose of a pre-determined quantity of medication can be arranged; and a second housing part which is fixed substantially non-releasably to the first housing part, wherein the first and second housing parts are movable relative to one another between a first position wherein the cavities with powdered medication are closed and a second position in which the cavity is placed into connection with an air path and the medication can be inhaled.
GB 2 264 237 discloses a powder inhaler comprising an openable casing having a mouthpiece and containing a replaceable magazine disc provided with a plurality of recesses or cups, each holding a dose of powder and being individually closed by a discrete closure element which can be opened and then reclosed. The disc may be indexed around to present a closed cap adjacent a projection 16 at the mouthpiece and then moved in the casing transverse to its rotational axis so that the projection removes the closure element. A ring is provided in the top casing portion to assist reclosure of the closure element after inhalation.
US 6,006,747 discloses a dry powder inhaler having a lid pivotally attached to an inhaler housing. A medicine-containing cartridge is attached to the top of the housing and includes a cartridge ring with apertures for holding dry powdered medicine. A slide-groove assembly located on the underside of the lid advances the cartridge ring to a next aperture when a user first opens, then closes the lid. A venturi air passageway assembly is contained within the housing of the dry powdered inhaler. The venturi air passageway assembly includes a tapered inlet section, a throat section, and a tapered outlet section. A pressure switch is advantageously located within the housing for actuating the mixing process within the mixing chamber. During inhalation, the pressure switch contained within the housing actuates the motor driven impeller within the mixing chamber of the inhaler when the pressure within the venturi air passageway reaches a predetermined level. The pressure at which the pressure switch closes corresponds to a specifically calibrated inhalation flow rate.
EP 0 525 720 discloses an oral or nasal inhalation device comprises an air passageway optionally shaped as a venturi passing through a body. A holder on the body receives a single dose powder container containing a medicament. A piercer or needle having a bore therethrough extends from the body into the holder. The user places the container and removes a seal on the top end of the container. Alternatively, the piercer is longer than the container and pierces both ends of it, obviating the need for a removable seal. A sharp inhalation causes air to be drawn through the air intake, creating a partial vacuum in the air passageway and causing the powder to be dispensed from the container through the bore of the piercer. Another embodiment has two pierces, each acting on an end of the container. Further embodiments make use of a rotable disk provided with multiple containers.
US 6,065,472 discloses a powder inhalation device comprising a housing containing a pharmacologically active compound, a conduit with an outlet extending into the housing through which a user can inhale to create an airflow through the conduit, a dosing unit for delivering a dose of the compound to the conduit and baffles arranged within the said conduit to aid disintegration of powder agglomerates entrained in said airflow. The baffles comprise a plurality of staggered plates extending into the conduit from opposite sides of the conduit at an angle of less than 90° to the sides of the conduit and are inclined towards the outlet to create a plurality of constrictions within the conduit and a plurality of changes in the direction of the said airflow through the conduit.
US 5,458,135 discloses a device for accurately delivering aerosolized doses of a medicament disperses a measured amount of drug in a measured volume of carrier gas and transfers the resulting aerosol to a chamber priorto inhalation by a patient. The chamber is filled efficiently with the aerosol, and inhalation by the patient draws the aerosol dose into the lungs. This is followed by the inhalation of atmospheric air that will push the initial dose well into the lung interiors. The apparatus optimally includes a dose regulator, a counter, a clock, a dose memory and a signal to indicate when a dose is ready for inhalation. Optimal chamber designs are disclosed.
US 5,388,572 discloses a dry powder inhalator for delivering a precise dose of a medicament contains a mesh disc impregnated with a series of spaced, medicament doses about the disc periphery. The inhalator is armed by manually retracting a finger pull extending downwardly from the bottom of the inhalator housing or rotating the bottom of the housing. The user inserts a mouthpiece on the housing into the mouth and inhales. This causes a chamber in the housing under a diaphragm to evacuate, thereby pulling the diaphragm down onto a knock out lever. The pivoting of the lever enables release of a piston into a cylinder which first compresses, and then dispenses a reduced volume of air at high pressure in a burst up through the medicament disc. When the burst of air hits the impregnated disc, the dose is forced out of the mesh's interstice, producing a cloud of the drug in its powdered form, which is inhaled by the user.
US 5, 622, 166 discloses a powder storage and delivery system for a drug powder inhaler has a carrier disk with a blister shell sealed by a shear layer. A tab is adhered to the shear layer, underneath the blister shell. The carrier disk is placed into a dry powder inhaler. An actuator pushes against the tab, causing the shear layer to tear away, releasing the powder drug contents from the blister into the dry powder inhaler. A disk carrier has bursting blisters with a brittle blister shell sealed with a foil lid, and covered by a plate. An actuator moves against the plate, causing the plate to buckle and the blistershell to burst open, releasing powdered drug into the dry powder inhaler.

### Summary of the Invention

The present disclosure accordingly provides a pre-metered dose assembly for consistently supplying precise doses of medicament to a breath-actuated dry powder inhaler as set forth in claim 1. The assembly includes a cap defining a dry powder delivery passageway for providing air to a dry powder supply port of a swirl chamber of a breath-actuated dry powder inhaler, and a magazine including a plurality of reservoirs for holding pre-metered doses of dry powder. One of the magazine and the cap is movable with respect to the other of the magazine and the cap for sequentially positioning the reservoirs within the delivery passageway of the cap. A breath-induced low pressure at an outlet port of the swir1 chamber of the inhaler causes an air flow through the dry powder delivery passageway of the assembly and into the dry powder supply port of the swir1 chamber. The air flow entrains dry powder from the reservoir positioned in the passageway for inhalation by a patient using the inhaler.

The present disclosure also provides a breath-actuated dry powder inhaler including the pre-metered dose assembly in combination with a de-agglomerator for breaking up aggregates and micronizing particles of dry powder prior to inhalation of the powder by a patient. The de-agglomerator includes an inner wall defining a swirl chamber extending along an axis from a first end to a second end, a dry powder supply port, one or more primary air flow inlet ports, and an outlet port. The supply port is at the first end of the swirl chamber for providing fluid communication between the dry powder deliver passageway of the pre-metered dose assembly and the first end of the swirl chamber. The primary airflow inlet ports are in the inner wall of the swirl chamber adjacent to or near the first end of the swirl chamber and provide fluid communication between a region exterior to the de-agglomerator and the swirl chamber. The outlet port provides fluid communication between the second end of the swirl chamber and a region exteriorto the de-agglomerator.

A breath-induced low pressure at the outlet port of the de-agglomerator causes air flows into the swirl chamber through the dry powder supply port and the inlet port. The air flows collide with each other and with the wall of the swirl chamber prior to exiting through the outlet port, such that any powder entrained in the air flows is broken down and micronized. The de-agglomerator further includes vanes at the first end of the swirl chamber for creating additional collisions and impacts of entrained powder.

Further features and advantages of the presently disclosed pre-metered dose magazine will become more readily apparent so those having ordinary skill in the art to which the present disclosure relates from the following detailed description and attached drawings.

### Brief Description of the Drawings

So that those having ordinary skill in the art will more readily understand how to construct a pre-metered dose magazine and a breath-actuated, dry powder inhaler in accordance with the present disclosure, a preferred embodiments are described in detail below with reference to the drawing figures wherein:
FIG. 1A is a top isometric view of a breath-actuated, dry powder inhaler including a pre-metered dose magazine according to the present disclosure;
FIG. 1B is a sectional view of the inhaler of FIG. 1A;
FIG. 2 is a top isometric view of the inhaler of FIG. 1A with a cap of the inhaler removed;
FIG. 3 is a top isometric view of the inhaler of FIG. 1A with the cap and the pre-metered dose magazine removed to reveal a de-agglomerator of the inhaler including a cover and a base;
FIG. 4 is a top isometric view of the base of the inhaler of FIG. 1A;
FIG. 5 is an exploded, bottom isometric view of the inhaler of FIG. 1A;
FIG. 6 is an enlarged bottom plan view of a portion of the cap of the inhaler of FIG. 1A;
FIG. 7 is an exploded, top isometric view of the cap and the pre-metered dose magazine of the inhaler of FIG. 1A;
FIG. 7a is a top isometric view of an alternative pre-metered dose magazine not in accordance with the invention for use with the inhaler of FIG. 1A;
FIG. 8 is a sectional view of portions of the cap and thepre-metered dose magazine of the inhaler of FIG. 1A;
FIG. 9 is a sectional view of the inhaler of FIG. 1A illustrating operation of the inhaler; and
FIG. 10 is an exploded, top isometric view of an additional breath-actuated, dry powder inhaler according to the present disclosure.

### Description of the Preferred Embodiment

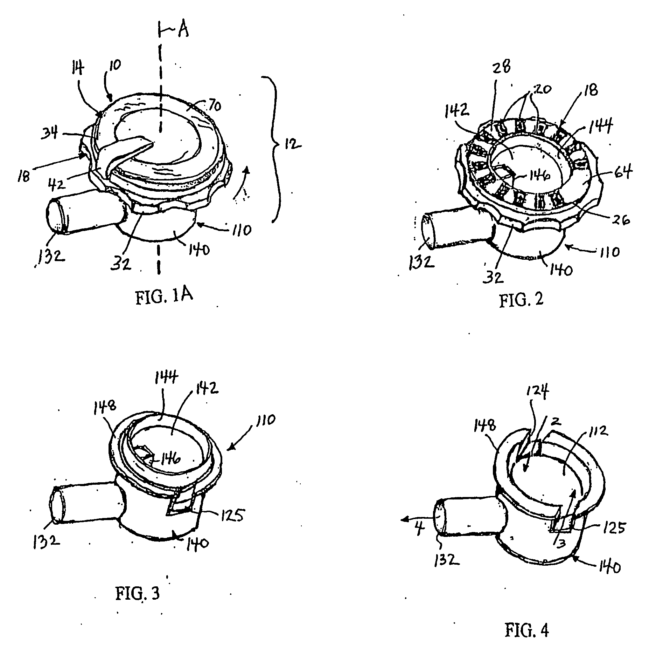

FIGS. 1A, 1B, 5 and 9 show a preferred embodiment of a pre-metered dose assembly 10 in a dry powder inhaler and, in particular, a breath-actuated, dry powder inhaler 12, all in accordance with the present disclosure. The pre-metered dose assembly 10 consistently furnishes precise doses of dry powder, e.g., a dry powder medicament or medicament composition, for inhalation by a patient using the dry powder inhaler 12.

The inhaler 12 generally includes the assembly 10, a swirl chamber 114 extending along axis A, a dry powder supply port 122 in a first end 118 of the swirl chamber, and an outlet port 132 at a second end 120 of the swirl chamber. The assembly 10 includes a cap 14 defining a dry powder delivery passageway 16 for providing air to the dry powder supply port 112 of the swirl chamber 114, and a magazine 18 including a plurality of reservoirs 20 for holding pre-metered doses of dry powder.

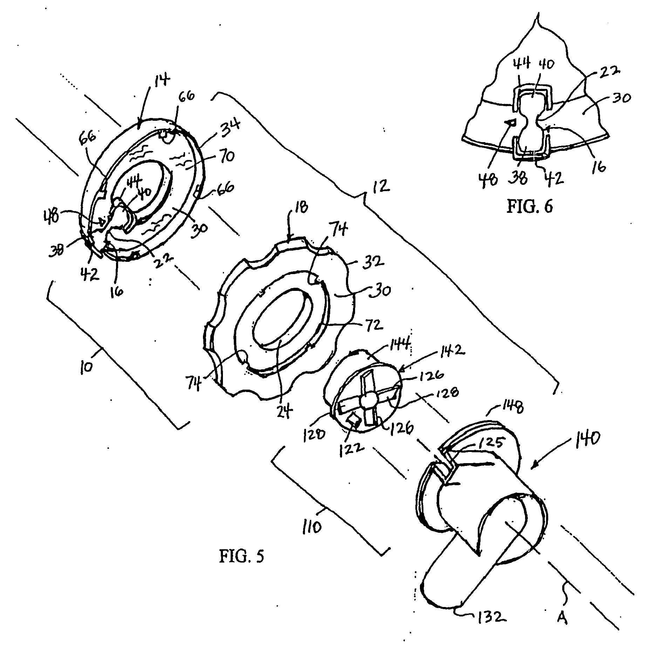

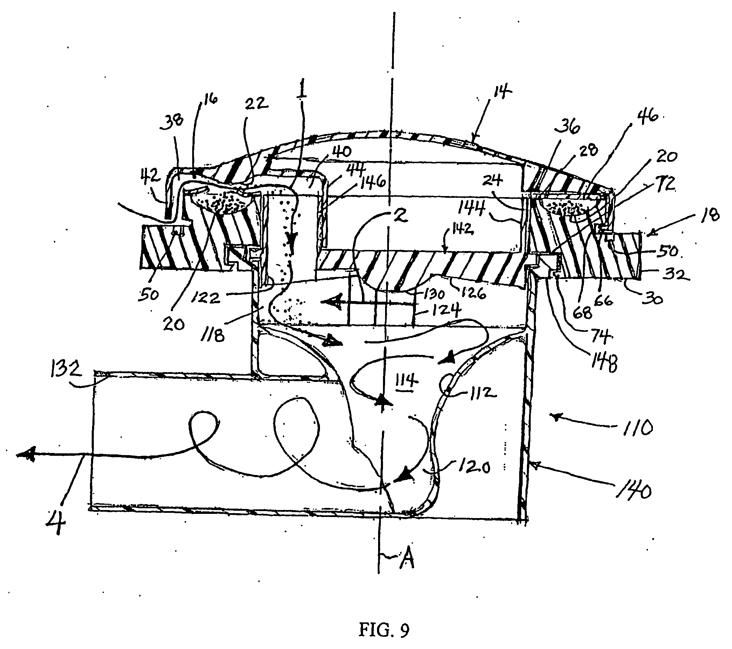

During operation, one of the magazine 18 and the cap 14 is movable with respect to the other of the magazine and the cap for sequentially positioning the reservoirs 20 of the magazine 18 within the delivery passageway 16 of the cap 14. Then, a breath-induced low pressure at the outlet port 132 of the swirl chamber 114 of the inhaler 12 causes an air flow, as indicated by arrow 1 in FIG. 9, through the dry powder delivery passageway 16 into the dry powder supply port 122 of the swirl chamber 114. As shown best in FIGS. 5, 6 and 9, the passageway 16 of the cap 14 includes a venturi 22 (or venturitype restriction) that causes the velocity of the breath-induced air flow to increase. The air pressure in the venturt 22 decreases as a result of the increased velocity, and the drop in pressure causes the pre-metered dose of dry powderto be dragged, or entrained into the air flow traveling to the swirl chamber 114.

The magazine 18 is movable with respect to the cap 14 for sequentially positioning the dry powder reservoirs 20 of the magazine 18 within the delivery passageway 16 of the cap 14. However, it should be understood that the magazine 18 could be made stationary, and the cap 14 made moveable with respect to the magazine 18 for sequentially positioning the passageway 16 over the reservoirs 20.

As shown in FIGS. 1A, 1B, 2, 5, 7 and 9, the magazine 18 is provided with an annular shape such that rotation of the annular magazine 18 sequentially positions the plurality of the dry powder reservoirs 20 within the delivery passageway 16 of the cap 14. However, it should be understood that the magazine 18 could be provided in other, suitable shapes and the cap 14 suitably adapted. For example, the magazine 18 could be provided with a straight elongated shape, such that movement of the magazine in the direction of elongation sequentially positions the reservoirs 20 within the delivery passageway 16 of the cap 14.

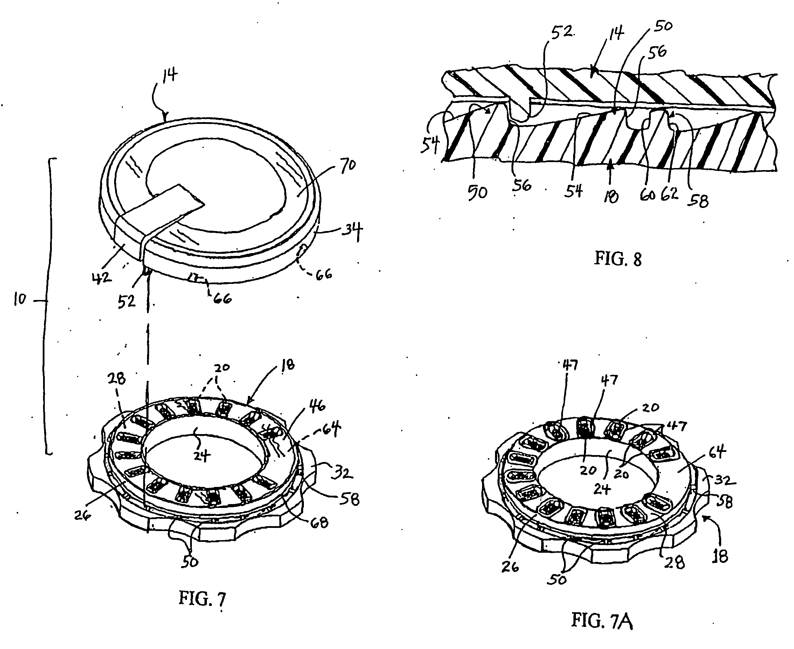

In particular, the annular magazine 18 includes inner and outer circumferencial surfaces 24, 26, and flat top and bottom annular surfaces 28, 30. The magazine 18 also includes a dial 32 radially extending outwardly from the outer circumferential surface 26 for allowing a patient to grip and rotate the magazine 18. The dry powder reservoirs 20 are provided in the top surface 28 of the magazine 18 and are uniformly sized and spaced with respect to one another, as shown best in FIGS 2 and 7.

As shown in FIGS. 1A, 1B, 5, 7 and 9, the cap 14 is circular and includes a cylindrical side wall 34 received on the outer circumferential surface 26 of the magazine 18, and a flat, bottom annular surface 36 received over the annular top surface 28 of the magazine 18. The magazine 18 and the cap 14, therefore, are adapted for rotation of the magazine 18 within the cap 14. As shown best in FIGS. 5, 6 and 9, the bottom surface 36 of the cap 14 defines the dry powder delivery passageway 16, which extends radially inwardly from a first end 38 at the side wall 34 of the cap 14, to a second end 40 at an inner circumference of the annular bottom surface 36 of the cap 14. The cap 14 also includes a first hood 42 extending downward from the first end 38 of the delivery passageway 16, and creating an air inlet port to the passageway 16 between the cap 14 and the magazine 18. A second hood 44 extends downward from the second end 40 of the delivery passageway 16, into the central void of the annular magazine 18.

The assembly 10 includes a seal for sealing the doses of dry powder in the reservoirs 20 of the magazine 18 in an airtight manner prior to the reservoirs 20 being positioned within the delivery passageway 16 of the cap 14. As shown best in FIGS. 7 and 9, the seal comprises a thin plastic film 46 secured to the annular top surface 28 of the magazine 18 and covering the dry powder in the reservoirs 20 in an airtight manner. The cap 14 includes means for piercing the film 46 above each of the reservoirs 20 prior to the reservoirs 20 being positioned within the delivery passageway 16 of the cap 14. As shown best in FIGS. 5 and 6, the means for piercing comprises a small barb 48 extending downward from the annular bottom surface 36 of the cap 14 in front of the venturi 22 of the delivery passageway 16 (assuming a counter-clockwise rotation of the magazine 18 with respect to the cap 14).

It is intended that a manufacturer will fill the reservoirs 20 of the magazine 18 with properly metered individual doses of dry powder medicament, or medicament composition including medicament and a suitable particulate carrier such as lactose. The filled reservoirs 20 are then sealed in an airtight manner, with the film 46 for example, and the magazine 18 and the cap 14 are provided as an assembly 10 to patients for use with a breath actuated, dry powder inhaler. The pre-metered dose assembly 10 may be provided as part of a disposable inhaler. Alternatively, the dose assembly 10 may be removably insertable into a non-disposable inhaler so that an empty assembly can be replaced by a full assembly.

Referring to FIG. 7A, a seal for sealing the doses of dry powder in the reservoirs 20 in an airtight manner can alternatively comprise continuous seals 47 surrounding each reservoir on the top surface 28 of the magazine 18. Each seal 47 is made from a soft resilient material, such as a synthetic rubber, and is raised slightly above the level of the top surface 28 of the magazine 18 so that the seal 47 is compressed between the bottom surface 30 of the cap 14 and the top surface 28 of the magazine 18. The compressed seals 47 retain the dry powder in the reservoirs 20 in an airtight manner prior to the reservoirs being moved into the delivery passageway 16. Means for piercing are not required. Preferably, the seals 47 are formed with the magazine 18 in a two step injection molding process.

Preferably, the magazine 18 and the cap 14 are movable with respect to each other through a plurality of discrete increments, wherein at each increment one of the plurality of the dry powder reservoirs 20 of the magazine 18 is positioned within the delivery passageway 16 of the cap 14. In addition, the magazine 18 and the cap 14 are preferably movable in a single direction only with respect to each other, so that a user can access the reservoirs in sequence, without being able to access one of the reservoirs more than once. Furthermore, movement between the magazine 18 and the cap 14 is preferably prevented after all the dry powder reservoirs 20 of the magazine 18 have been positioned in the delivery passageway 16 of the cover, to provide an indication to a patient that all of the doses of the magazine 18 have been used.

As shown best in FIGS. 7 and 8, one of the magazine 18 and the cap 14 includes a plurality of teeth 50, and the other of the magazine 18 and the cap 14 includes a resilient pawl 52 sequentially passing over the teeth during movement of the magazine 18 with respect to the cap 14. When the pawl 52 is between two of the teeth 50, a reservoir 20 of the magazine 18 corresponding to the two teeth is positioned in the delivery passageway 16 of the cap 14. Each of the plurality of teeth 50 has a sloped first side 54 allowing passage of the pawl 52 in only a first direction, and a straight second side 56 preventing passage of the pawl in a second direction. Accordingly, as shown, the magazine 18 can only be rotated in a counter-clockwise direction with respect to the cap 14. In addition, one tooth 58 has straight first and second sides 60, 62 that prevent passage of the pawl 52 past the tooth 58 in any direction. The "last" tooth 58 is positioned to correspond with an empty portion 64 of the top surface 28 of the magazine 18 to prevent movement between the magazine 18 and the cap 14 after all the reservoirs 20 of the magazine 18 have been rotated through the delivery passageway 16.

The assembly 10 also includes a coupler for securing the cap 14 to the magazine 18. As shown best in FIGS. 5 and 9, the coupler comprises resilient tangs 66 extending radially inward from a bottom edge of the side wall 34 of the cap 14 and engaging a circumferential groove 68 of the outer circumferential surface 26 of the magazine 18. The tangs 66 and the groove 68 prevent the cap 14 from being lifted off the magazine 18, yet allow the magazine 18 to rotate with respect to the cap 14.

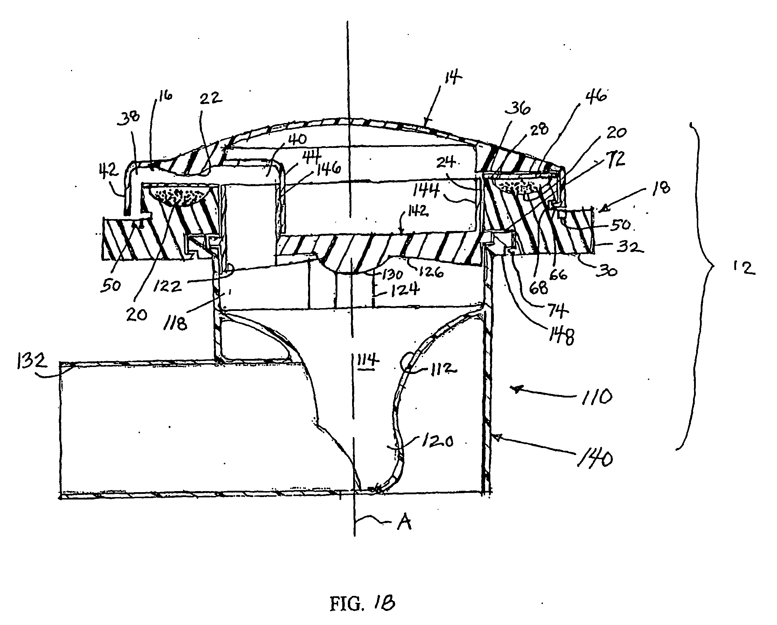

The assembly 10 additionally includes an indicator for indicating the number of dry powder reservoirs 20 containing dry powder, i.e., the number of pre-metered doses remaining in the magazine 18. As shown in FIGS. 1,5 and 7, the indicator comprises an annular transparent portion 70 of the cap 14, which allows the reservoirs 20 of the magazine 18 to be viewed through the cap 14 for a determination of how many of the reservoirs 20 contain medicament. Other suitable indicators could alternatively be provided. For example, sequential printed numbers corresponding to the reservoirs 20 of the magazine 18 can be provided on the dial 32 of the magazine 18, so that the number of reservoirs 20 that have passed through the delivery passageway 16 of the cap 14 can be determined by reference to the printed numbers.

Referring to FIGS. 1A through 4 and FIG. 9, the inhaler preferably includes both the presently disclosed pre-metered dose assembly and a de-agglomerator 110. The de-agglomerator 110 is disclosed in co-pending provisional U.S. patent application serial no. 60/213,382, filed June 23, 2000 (entitled "De-Agglomerator for Breath-Actuated Dry Powder Inhaler"). The co-pending application is assigned to the assignee of the present disclosure and has been incorporated herein by reference. As its name implies, the de-agglomerator 110 breaks down agglomerates of dry powder before inhalation of the dry powder by a patient.

In general, the de-agglomerator 110 includes an inner wall 112 defining the swirl chamber 114 extending along the axis A from the first end 118 to the second end 120 of the chamber. The swirl chamber 114 includes circular cross-sectional areas arranged transverse to the axis A, which decrease from the first end 118 to the second end 120 of the swirl chamber 114. Preferably, the cross-sectional areas of the swirl chamber 114 decrease monotonically such that any air flow traveling from the first end 118 of the swirl chamber 114 to the second end 120 will at least in part collide with the inner wall 112 of the chamber. In addiction, as shown best in FIGS. 1B and 9, the sidewall is preferably convex, i.e., arches inwardly towards the axis A.

Preferably, the dry powder supply port 122 of the de-agglomerator 110 faces in a direction substantially parallel with the axis A of the chamber 114. Accordingly, as shown in FIG. 9, the air flow 1 entering the chamber 114 through the supply port 122 is at least initially directed parallel with respect to the axis A of the chamber.

Referring to FIGS. 1B, 3, 4, 5 and 9, the de-agglomerator 110 additionally includes at least one inlet port 124 in the inner wall 112 of the swirl chamber 114 adjacent to the first end 118 of the chamber providing fluid communication between a region exterior to the de-agglomerator and the first end 118 of the swirl chamber 114. Preferably, the at least one inlet port comprises two diametrically opposed inlet ports 124, 125 that extend in a direction substantially transverse to the axis A and substantially tangential to the circular cross-section of the swirl chamber 114. As a result, air flows, illustrated by arrows 2 and 3 in FIGS. 4 and 9, entering the chamber 114 through the inlet ports 124, 125 are at least initially directed transverse with respect to the axis A of the chamber and collide with the air flow 1 entering through the supply port 122 to create a combined turbulence airflow illustrated by arrow 4.

Referring to FIGS. 1B, 5 and 9, the de-agglomerator 110 includes vanes 126 at the first end 118 of the swirl chamber 114 extending at least in part radially outwardly from the axis A of the chamber. Each of the vanes 126 has an oblique surface 128 facing at least in part in a direction transverse to the axis A of the chamber. The vanes 126 are sized such that at least a portion of the combined air flows 4 collide with the oblique surfaces 128. Preferably, the vanes comprise four vanes 126, each extending between a hub 130 aligned with the axis A and the wall 112 of the swirl chamber 114.

Referring to FIG. 9, the geometry of the swirl chamber 114 causes the combined air flows 4 and the entrained dry powder to follow a turbulent spiral path, or vortex, through the chamber. As will be appreciated, the decreasing cross-sections of the swirl chamber 114 continuously changes the direction and increases the velocity of the spiraling combined air flow 4 and entrained dry powder. Thus, particles and any agglomerates of the dry powder constantly impact against the wall 112 of the swirl chamber 114 and collide with each other, resulting in a mutual grinding or shattering action between the particles and agglomerates. In addition, particles and agglomerates deflected off the oblique surfaces 128 of the vanes 126 cause further impacts and collisions. The constant impacts and collisions cause any agglomerates of dry powder to break into additional particles, and cause the particles to be substantially micronized.

Upon exiting the swirl chamber 114, the direction of the combined air flow 14 and the entrained dry powder is again changed to a transverse direction with respect to the axis A, through the outlet port 132. The combined air flow 4 and the entrained dry powder retain a swirl component of the flow, such that the air flow 4 and the entrained dry powder spirally swirls through the outlet port 132. Since the micronized powder and any remaining agglomerates maintain the swirl imparted from swirl chamber 114, the swirling flow causes additional impacts in the outlet port 132 so as to result in further breaking up of any remaining agglomerates prior to being inhaled by a patient. The de-agglomerator 110, therefore, ensures that particles of the dry powder are small enough for adequate penetration of the powder into a bronchial region of a patient's lungs during inhalation.

As shown best in FIGS. 1B, 3, 4, 5 and 9, the de-agglomerator 110 is preferably assembly from two pieces: a cup-like base 140 and a cover 142. The base 140 and the cover 142 are connected to form the swirt chamber 114. The cup-like base 140 includes the wall 112 and the second end 120 of the chamber and defines the outlet port 132. The base 140 also includes the inlet ports of the swirl chamber 114. The cover 142 forms the vanes 128 and defines the supply port 122.

As shown best in FIGS. 1B, 2, 3, 5 and 9, the cover 142 includes an upwardly extending cylindrical guide 144, and a chimney 148 extending upwardly from the supply port 122 within the guide. The inner circumference 24 of the annular magazine 18 is received coaxially on the guide 144, such that the magazine can be rotated about the guide. The bottom surface 30 of the magazine 18 includes an annular recess 72 receiving a rim 148 of the base 140. The second hood 44 of the cap 14 is received over the chimney 146 of the supply port 122 to connect the delivery passageway 16 of the cap 14 with the supply port 122 of the de-agglomerator 110. In addiction, the inhaler 12 includes a coupler for securing the pre-metered dose assembly 10 to the de-agglomerator 110, such that the magazine 18 is free to be rotated with respect to the de-agglomerator. As shown best in FIGS. 1B, 5 and 9, the coupler comprises resilient tangs 74 of the magazine 18 engaging a bottom surface of the rim 148 of the base 140, preventing the assembly 10 from being lifted off the de-agglomerator 110 yet allowing the magazine 18 to rotate.

The base 140, the cover 142, the magazine 18 and the cap 14 are preferably manufactured from a plastic such as polypropylene, acetal or moulded polystyrene, but may be manufactured from metal or another suitable material. Preferably, the cover 142 includes an anti-static additive, such that the dry powder will not cling to the vanes 128. The base 140 and the cover 142 are connected in a mannerthat provides an air tight seal between the parts. For this purpose heat or cold sealing, laser welding or ultrasonic welding could be used, for example.

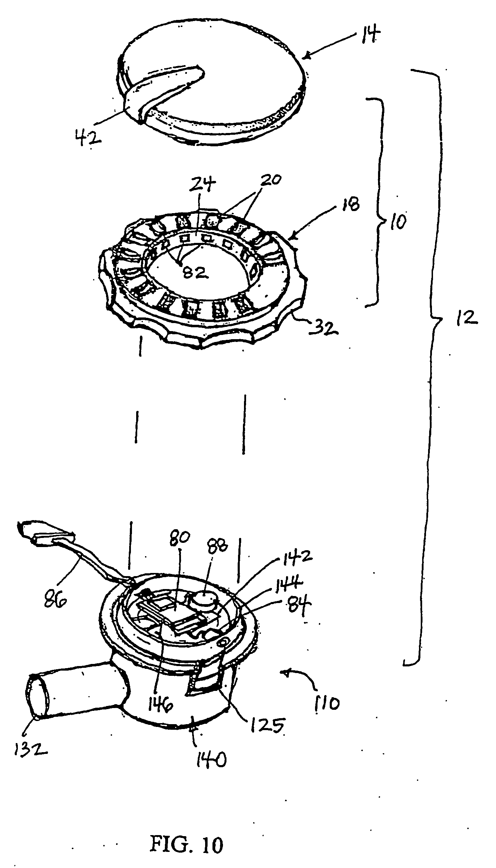

Referring now to FIG. 10, an inhaler 12 according to the present disclosure can be provided with a processor 80 for recording how many doses are inhaled from the inhaler by a patient, and at what time the doses are inhaled. The inhaler 12 includes indicators 82 attached to the magazine 18 corresponding to the dry powder reservoirs 20, and a detector 84 mounted on the de-agglomerator 110. The detector 84 provides a signal when one of the indicators 82 passes the detector as the magazine 18 is rotated with respect to the de-agglomerator 110. A signal from the detector 84, therefore, is indicative of a single dose of dry powder being inhaled by a patient through the inhaler 12. The indicators can comprise, for example, reflective strips, while the detector can comprise an LED for directing light on passing reflective strip and a receiver for receiving the reflected light.

Although not shown, a counter provides a sum of the number of signals provided by the detector, while a clock provides a chronological time for each signal provided by the detector. The processor 80 then provides predetermined calculations based upon the sum provided by the counter and the chronological times provided by the dock. The calculations might comprise, for example, the number of doses inhaled by a patient over a day, week or month. A memory stores the calculations provided by the processor 80, and the inhaler 12 further includes a transmitter 86 for transmitting the stored calculations to a remote device for utilizing the calculations. The transmitter might comprise a cable 86 for connection to a doctor's computer upon a patient's visit to the doctor's office, for example. The inhaler 12 includes a battery 88 for powering the detector 84 and the processor 80.

It should be understood that the foregoing detailed description and preferred embodiment is only illustrative of a breath-actuated dry powder inhaler 12 according to the present disclosure. Various alternatives and modifications to the presently disclosed inhaler 12 can be devised by those skilled in the art without departing from the scope of the appended claims. For example, the pre-metered dose assembly 10 can be modified for with any inhaler and, in particular, any breath-actuated dry powder inhaler.

## Claims

1. A pre-metered dose assembly (10) for use with a breath-actuated dry powder inhaler(12), comprising:
a cap (14) defining a dry powder delivers passageway (16) for providing air to a dry powder supple port(112) of a chamber (114) of a breath-actuated dry powder inhaler (12); and
a magazine (18) including a plurality of reservoirs (20) for holding pre-metered doses of dry powder, does of dry powder being contained in the reservoirs of the magazine, one of the magazine (18) and the cap (14) movable with respect to the other of the magazine (18) and the cap (14) for sequentially positioning the reservoirs (20) within the delivery passageway (16) of the cap (14), and a seal (46) sealing each of the reservoirs (20) of the magazine (18) in an airtight manner prior the reservoir (20) being positioned within the delivery passageway (16) of the cap (14), wherein the seal (46) comprises a film secured to the magazine and covering the reservoirs (20) in an airtight manner
whereby a breath-induced low pressure in the chamber (114) of the inhaler (12) causes an air flow through the dry powder delivery passageway (16) and into the dry powder supply port (122), and the air flow entrain dry powder from the dry powder reservoir (20) positioned in the passageway (16) into the chamber for inhalation by a patient using the inhaler (12);
and **characterised in that** the cap further comprises means (48) for piercing the film (46) above each of the reservoirs (20) prior to the reservoir (20) being positioned within the delivery passageway (16) of the cap (14).

2. An assembly according to claim 1, wherein the magazine (18) is movable with respect to the cap (14) for sequentially positioning the plurality of the dry powder reservoirs (20) within the delivery passageway (16) of the cap (14).

3. An assembly according to claim 2, wherein the magazine (18) is annular such that rotation of the annular magazine (18) sequentially positions the plurality of the dry powder reservoirs (20) within the delivery passageway (16) of the cap (14).

4. An assembly according to any one of claims 1 to 3, further including means for indicating the number of dry powder reservoirs containing dry powder.

5. An assembly according to claim 4, wherein the cap (14) covers the plurality of the dry powder reservoirs (20) of the magazine (18) and the means for indicating comprises a transparent portion (70) of the cap (14).

6. An assembly according to any one of claims 1 to 5, wherein one of the magazine (18) and the cap (14) is movable with respect to the other of the magazine and the cap through a plurality of discrete increments, wherein at each increment one of the plurality of the dry powder reservoirs (20) is positioned within the delivery passageway (16) of the cap (14).

7. An assembly according to any one of claims 1 to 6, wherein one of the magazine (18) and the cap (14) is movable with respect to the other of the magazine and the cap in only a single direction.

8. An assembly according to any one of claims 1 to 7, adapted such that the magazine (18) and the cap (14) will be unmovable with respect to each other after all of the dry powder reservoirs (20) of the magazine (18) have been positioned in the dry powder delivery passageway (16) of the cover (142).

9. An assembly according to any one of claims 1 to 8, wherein:
one of the magazine (18) and the cap (14) includes a plurality of teeth (50); and
the other of the magazine (18) and the cap (14) includes a resilient pawl (52) sequentially passing over the teeth (50) during movement of one of the magazine (18) and the cap (14) with respect to the other of the magazine (18) and the cap (14), wherein the magazine (18) is in one of the plurality of discrete increments when the pawl (52) is between teeth (50).

10. An assemble according to claim 9, wherein each of the plurality of teeth (50) has a sloped first side (54) allowing passage of the pawl (52) in a first direction, and a straight second side (56) preventing passage of the pawl in a second direction.

11. An assembly according to claim 9, wherein the plurality of teeth (50) includes one tooth (58) having straight first and second sides preventing passage of the pawl (52) past said one tooth (58).

12. An assembly according to any preceding claim, wherein the film (46) secured to the magazine (18) and covering the dry powder in the reservoirs in an airtight manner comprises a plastic film.

13. An assembly according to nay one of claims 1 to 12, wherein the dry powder delivery passageway (16) of the cap includes a venturi (22).

14. An assembly according to any one of claims 1 to 13, wherein:
the magazine (18) is annular such that rotation of one of the magazine (18) and the cap (14) with respect to the other of the magazine and the cap sequentially positions the plurality of the dry powder reservoirs (20) within the delivery passageway (16) of the cap (14), the magazine (18) having a top surface (28) defining the reservoirs (20); and
the cap (14) includes a lower surface (36) received over the top surface (28) of the magazine, the lower surface (36) defining the dry powder delivery passageway (16) extending radially inwardly from an other portion to an inner portion of the cap (14).

15. An assembly according to any one of claims 1 to 14, further comprising means (66, 68) for securing the cap (14) to the magazine (18).

16. An assembly according to any one of claims 1 to 15, further comprising means (74, 148) for securing the assembly (10) to a chamber (110) of an inhaler (12).

17. A breath-actuated dry powder inhaler (12) including an assembly (10) according to any one of claims 1 to 16 and further comprising:
a chamber (114) extending along an axis (A) between a first end (118) and a second end (120);
a dry powder supply port (122) in the first end (118) of the chamber (114) in fluid communication with the dry powder delivery port (16) of the cap (14); and
an outlet port (132) at a second end of the chamber (120);
whereby a breath-induced low pressure at the outlet port (132) causes airflow into the chamber (114) through the dry powder supply port (122), with the air flow through the dry powder supply port (122) entraining dry powder into the chamber(120) from the reservoir(20) of the magazine (18) positioned in the delivery passageway (16).

18. An inhaler according to claim 17, further comprising a de-agglomerator (110) having:
an inner wall (112) defining the chamber (114) extending along an axis (A) from a first end (118) to a second end (120);
at least one inlet port (124) in the inner wall (112) adjacent to the first end (118) of the chamber (114) providing fluid communication between a region exterior to the de-agglomerator and the first end (118) of the chamber (114);
whereby a breath-induced low pressure at the outlet port (132) also causes an air flow into the chamber (114) through the inlet port (124,125).

19. An inhaler according to claim 18, wherein the de-agglomerator (110) further comprises vanes (126) at the first end (118) of the chamber (114) extending at least in part radially outwardly from the axis (A) of the chamber (114), each of the vanes (126) having an oblique surface (128) facing at least in part in a direction transverse to the axis (A).

20. An inhaler according to any one of claims 18 and 19, wherein the chamber (114) of the de-agglomerator (110) includes cross-sectional areas arranged transverse to the axis (A), the cross-sectional areas decreasing monotonically from the first end (118) to the second end (120) of the chamber (114).

21. An inhaler according to any one of claims 18 to 20, wherein:
the dry powder supply port (112) of the de-agglomerator (110) faces in a direction substantially parallel to the axis(A);
the outlet port (132) extends substantially transverse to the axis (A); and
the at least one inlet port (124, 125) extends in a direction substantially transverse to the axis (A) and substantially tangential to the chamber (114).

22. An inhaler according to claim 21, wherein the at least one inlet port (124, 125) of the de-agglomerator (110) comprises two diametrically opposed inlet ports (124 and 125).

23. An inhaler according to any one of claims 18 to 22, wherein:
the de-agglomerator (110) comprises a cup-like base (140) closed with a cover (142) to form the chamber (114), the base (140) defining the inner wall (112), and the second end (120) of the chamber (114) and the outlet port (132), the cover (142) defining the first end (118) of the chamber (114), the vanes (126) and the supply port (122), and the base (140) and the cover (142) in combination defining the at least one inlet port (124,125), the cover (142) including a cylindrical guide (144) extending upwardly from the chamber and a chimney (146) extending upwardly within the cylindrical guide from the supply port (122);
the magazine (18) is annular and has an inner circumferential surface (24) received coaxially on the cylindrical guide (144) of the de-agglomerator (110) for rotation of the magazine (18) with respect to the de-agglomerator (110), the magazine (18) having a top surface (28) defining the dry powder reservoirs (20); and
the cap (14) includes a lower surface (36) received over the top surface (28) of the magazine (18), the lower surface (34) defining the dry powder delivery passageway (16) extending radially inwardly from an outer portion to an inner portion of the cap (14), the cap (14) further including a hood (44) extending downwardly from the inner portion and received over the chimney (146) of the de-agglomerator (110), the hood (44) connecting the delivery passageway (16) to the chimney (146) and preventing rotation of the cap (14) with respect to the de-agglomerator (110).

24. An inhaler according to any one of claims 18 to 23, further comprising:
indicators (82) attached to the magazine (18) corresponding to the dry powder reservoirs (20); and
a detector (84) mounted on the de-agglomerator (110) providing a dose signal upon an indicator (82) passing the detector (84) as the magazine (18) is moved with respect to the de-agglomerator (110) to position one of the reservoirs (20) in the dry powder delivery passageway (16), whereby a dose signal from the detector (84) is indicative of the dispensing of a single reservoir of dry powder through the inhaler (12).

25. An inhaler according to claim 24, further comprising:
a counter (not shown) providing a sum of the number of dose signals provided by the detector (84);
a clock for providing a time for each dose signal provided by the detector (84);
a processor (80) for providing predetermined calculations based upon the sum provided by the counter and the times provided by the clock; memory for storing the calculations provided by the 5 processor (80); and
means (86) for transmitting the stored calculations to a remote device for utilizing the calculations.

26. An assembly or inhaler according to any preceding claim, wherein each reservoir (20) of the magazine (18) contains a single dose of the dry powder.

27. An assembly or inhaler according to any preceding claim, wherein the dry powder comprises a medicament composition having at least one active agent medicament adhered to a particulate carrier.

## Patentansprüche

1. Eine vordosierte Spendereinheit (10) für einen atemaktivierten Trockenpulverinhalator (12) wie folgt:
bestehend aus einem Deckel (14) zur Definition eines Trockenpulver-Zuführkanals (16), der einem Trockenpulver-Zuführanschluss (122) in einer Kammer (114) eines atemaktivierten Trockenpulverinhalators (12) Luft zuführt; und
einem Magazin (18) mit diversen Behältern (20) für vordosierte Trockenpulverdosen, wobei die Trockenpulverdosen in den Behältern des Magazins enthalten sind, wobei das Magazin (18) und der Deckel (14) jeweils in Beziehung zum Magazin (18) und Deckel (14) beweglich sind, so dass die Behälter (20) nacheinander in den Zuführkanal (16) des Deckels (14) bewegt werden und eine Dichtung (46) jeden der Behälter (20) des Magazins (18) luftdicht abdichtet, bevor der Behälter (20) in den Zuführkanal (16) des Deckels (14) verschoben wird, wobei die Dichtung (46) eine Folie umfasst, die am Magazin befestigt ist und die Behälter (20) luftdicht abschließt;
bei dem ein atemaktivierter Niederdruck in der Kammer (114) des Inhalators (12) dazu führt, dass Luft durch den Trockenpulver-Zuführkanal (16) in den Trockenpulver-Zuführanschluss (122) geleitet wird und der Luftstrom Trockenpulver aus dem Trockenpulverbehälter (20) im Kanal (16) mitträgt und in die Kammer zur Inhalation durch den den Inhalator (12) benutzenden Patienten transportiert;
dadurch charakterisiert, dass der Deckel darüber hinaus Funktionen (48) bietet, um die Folie (46) über jedem Behälter (20) vor dem Positionieren des Behälters (20) im Zuführkanal (16) des Deckels (14) zu perforieren.

2. Eine Spendereinheit gemäß Patentanspruch 1, bei der das Magazin (18) in Bezug auf den Deckel (14) beweglich ist, so dass die Trockenpulverbehälter (20) nacheinander in den Zuführkanal (16) des Deckels (14) geleitet werden können.

3. Eine Spendereinheit gemäß Patentanspruch 2, bei der das Magazin (18) ringförmig ist, so dass die Drehung des ringförmigen Magazins (18) die Trockenpulverbehälter (20) nacheinander in den Zuführkanal (16) des Deckels (14) leitet.

4. Eine Spendereinheit gemäß einem der Patentansprüche 1 bis 3, die zudem einen
Mechanismus zur Anzeige der Anzahl von Trockenpulverbehältern mit Trockenpulver besitzt.

5. Eine Spendereinheit gemäß Patentanspruch 4, bei der der Deckel die Trockenpulverbehälter (20) des Magazins (18) bedeckt und der Anzeigemechanismus einen transparenten Bereich (70) im Deckel (14) umfasst.

6. Eine Spendereinheit gemäß einem der Patentansprüche 1 bis 5, bei der das Magazin (18) oder der Deckel (14) jeweils in Beziehung zum Magazin (18) und Deckel (14) in diversen Einzelschritten beweglich sind, wobei bei jedem Schritt einer der zahlreichen Trockenpulverbehälter (20) in den Zuführkanal (16) des Deckels (14) bewegt wird.

7. Eine Spendereinheit gemäß einem der Patentansprüche 1 bis 6, bei der das Magazin (18) oder der Deckel (14) jeweils in Beziehung zum Magazin (18) und Deckel (14) nur in einer Richtung beweglich sind.

8. Eine Spendereinheit gemäß einem der Patentansprüche 1 bis 7, die so angepasst ist, dass das Magazin (18) und der Deckel (14) in Beziehung zueinander nach Zuführung aller Trockenpulverbehälter (20) aus dem Magazin (18) in den Trockenpulver-Zuführkanal (16) des Deckels (14) nicht mehr beweglich sind.

9. Eine Spendereinheit gemäß einem der Patentansprüche 1 bis 8, bei der: das Magazin (18) oder der Deckel (14) zahlreiche Zähne (50) aufweisen; und das jeweils andere Bauteil - Magazin (18) oder Deckel (14) - mit einer elastischen Klinke (52) versehen sind, die bei Bewegung des Magazins (18) und des Deckels (14) zueinander über die Zähne (50) fährt, wobei sich das Magazin (18) in einem der Einzelschritte befindet, wenn die Klinke (52) zwischen zwei Zähnen (50) einrastet.

10. Eine Spendereinheit gemäß Patentanspruch 9, bei der jeder der Zähne (50) auf der einen Seite schräg zuläuft (54), so dass die Klinke (52) in eine Richtung darüber fahren kann, und auf der anderen Seite (56) gerade abfällt, um zu verhindern, dass sich die Klinke zurückbewegt.

11. Eine Spendereinheit gemäß Patentanspruch 9, bei der einer der Zähne (58) in der Gesamtmenge der Zähne (50) zwei gerade abfallende Kanten aufweist um zu verhindern, dass sich die Klinke über diesen Zahn (58) hinausbewegt.

12. Eine Spendereinheit gemäß einem der vorausgehenden Patentansprüche, bei der die Folie (46), die am Magazin (18) angebracht ist und das Trockenpulver in den Behältern luftdicht einschließt, aus Kunststoff besteht.

13. Eine Spendereinheit gemäß einem der Patentansprüche 1 bis 12, bei der der Trockenpulver-Zuführkanal (16) des Deckels mit einer Düse (22) versehen ist.

14. Eine Spendereinheit gemäß einem der Patentansprüche 1 bis 13, bei der:
das Magazin (18) ringförmig gestaltet ist, so dass die Drehung des Magazins (18) oder des Deckels (14) in Beziehung zueinander die Trockenpulverbehälter (20) nacheinander in den Zuführkanal (16) des Deckels (14) bewegt, wobei die Oberseite (28) des Magazins (18) die Form der Behälter (20) bestimmt und die Unterseite (30) des Deckels (14) über der Oberseite (28) des Magazins den Trockenpulver-Zuführkanal (16) definiert, indem sie strahlenförmig vom Deckeläußeren zum Inneren des Deckels (14) verläuft.

15. Eine Spendereinheit gemäß einem der Patentansprüche 1 bis 14 mit zusätzlichen Funktionen (66, 68), um den Deckel (14) auf dem Magazin (18) zu befestigen.

16. Eine Spendereinheit gemäß einem der Patentansprüche 1 bis 15 mit zusätzlichen Funktionen (74, 148), um die Spendereinheit (10) an einer Kammer (110) des Inhalators (12) zu befestigen.

17. Ein atemaktivierter Trockenpulver-Inhalator (12) mit einer Spendereinheit (10) gemäß einem der Patentansprüche 1 bis 16 und zusätzlich:
einer Kammer (114) entlang einer Achse (A) zwischen dem ersten Ende (118) und dem zweiten Ende (120);
einer Trockenpulver-Zuleitung (122) im ersten Ende (118) der Kammer (114) in beständiger Verbindung zum Trockenpulver-Zuführanschluss (16) des Deckels und einer Ausstoßöffnung (132) am anderen Ende der Kammer (120);
wobei ein atemaktivierter Niederdruck an der Ausstoßöffnung (132) dazu führt, dass Luft durch die Trockenpulver-Zuleitung (122) Trockenpulver aus dem Behälter (20) des Magazins (18) im Zuführkanal (16) in die Kammer (120) leitet.

18. Ein Inhalator gemäß Patentanspruch 17 zusätzlich mit einem Zerkleinerer (110) wie folgt:
einer Innenwand (112), die die Kammer (114) entlang der Achse (A) zwischen dem ersten Ende (118) und dem zweiten Ende (120) festlegt;
mindestens einem Zugang (124) in der Innenwand (112) neben dem ersten Ende (118) der Kammer (114) mit beständiger Verbindung zwischen einem Bereich außerhalb des Zerkleinerers und dem ersten Ende (118) der Kammer (114);
wobei ein atemaktivierter Niederdruck an der Ausstoßöffnung (132) auch zu einem Luftstrom durch den Zugang (124, 125) in die Kammer (114) führt.

19. Ein Inhalator gemäß Patentanspruch 18, bei dem der Zerkleinerer (110) am einen Ende (118) der Kammer (114) mit Schaufeln (126) versehen ist, die zumindest teilweise von der Achse (A) der Kammer (114) nach außen verlaufen, wobei jede Schaufel (126) eine abgeschrägte Fläche (128) besitzt, die zumindest teilweise diagonal zur Achse (A) verläuft.

20. Ein Inhalator gemäß einem der Patentansprüche 18 oder 19, bei dem die Kammer (114) des Zerkleinerers (110) diagonal zur Achse (A) verlaufende Querschnittbereiche aufweist, die gleichmäßig vom ersten Ende (118) zum zweiten Ende (120) der Kammer (114) abnehmen.

21. Ein Inhalator gemäß einem der Patentansprüche 18 bis 20, bei dem:
der Trockenpulver-Zuführanschluss (112) des Zerkleinerers (110) weitgehend parallel zur Achse (A) verläuft;
die Ausstoßöffnung (132) weitgehend diagonal zur Achse (A) verläuft; und
mindestens ein Zugang (124, 125) weitgehend diagonal zur Achse (A) und tangential zur Kammer (114) verläuft.

22. Ein Inhalator gemäß Patentanspruch 21, bei dem mindestens ein Zugang (124, 125) des Zerkleinerers (110) zwei diametrisch gegenüberliegende Zugänge (124 und 125) besitzt.

23. Ein Inhalator gemäß einem der Patentansprüche 18 bis 22, bei dem:
der Zerkleinerer (110) aus einer tassenähnlichen Basis (140) besteht, die durch eine Abdeckung (142) geschlossen ist und so die Kammer (114) bildet;
die Basis (140) definiert die Innenwand (112), das zweite Ende (120) der Kammer (114) Und der Ausstoßöffnung (132), die Abdeckung (142) definiert das erste Ende (118) der Kammer (114), die Schaufeln (126) und den Zuführanschluss (122), und die Basis (140) und Abdeckung (142) definieren zusammen mindestens einen Zugang (124, 125), wobei die Abdeckung (142) mit einer zylindrischen Führung (144) aus der Kammer und ein Zylinder (146) in der zylindrischen Führung aus dem Zuführanschluss (122) nach oben führen;
das Magazin (18) ringförmig ist und eine innere Ringfläche (24) besitzt, die koaxial auf der zylindrischen Führung (144) des Zerkleinerers (110) sitzt und der Drehung des Magazins (18) in Bezug auf den Zerkleinerer (110) dient; die Oberseite (28) des Magazins (18) definiert die Trockenpulverbehälter (20);
und die Unterseite (36) des Deckels (14) auf der Oberseite (28) des Magazins (18) zu liegen kommt, wobei die Unterseite (34) des Trockenpulver-Zuführkanals (16) strahlenförmig vom Äußeren des Deckels (14) nach innen verläuft und eine Haube (44) vom Innenteil nach unten verläuft und über den Zylinder (146) des Zerkleinerers (110) ragt, wobei die Haube (44) den Zuführkanal (16) mit dem Zylinder (146) verbindet und eine Drehung des Deckels (14) in Beziehung zum Zerkleinerer (110) verhindert.

24. Ein Inhalator gemäß einem der Patentansprüche 18 bis 23, der zudem ausgestattet ist mit:
Anzeigen (82) am Magazin (18) für die Trockenpulverbehälter (20); und
einem Detektor (84) am Zerkleinerer (110), der ein Dosissignal an eine Anzeige (82) liefert, die die Bewegung des Magazins (18) in Beziehung zum Zerkleinerer (110) am Detektor (84) registriert, wenn ein Behälter (20) im Trockenpulver-Zuführkanal (16) positioniert wird; das Dosissignal vom Detektor (84) zeigt die Ausgabe einer Einzeldosis Trockenpulver durch den Inhalator (12) an.

25. Ein Inhalator gemäß Patentanspruch 24, der zudem ausgestattet ist mit:
einem Zähler (nicht abgebildet), der die Anzahl der vom Detektor (84) gelieferten Dosissignale zählt;
einer Uhr, die die Zeit jedes Dosissignals vom Detektor (84) misst;
einem Prozessor (80) für programmierte Berechnungen basierend auf der Summe des Zählers und der von der Uhr angezeigten Zeitdauer;
einem Speicher zur Speicherung der Prozessorrechnungen (80); und
Funktionen (86) zur Übertragung der gespeicherten Rechnungen auf ein Remote-Gerät zur Analyse.

26. Eine Spendereinheit oder ein Inhalator gemäß einem der vorstehenden Ansprüche, wobei jeder Behälter (20) des Magazins (18) eine Einzeldosis Trockenpulver enthält.

27. Eine Spendereinheit oder ein Inhalator gemäß einem der vorstehenden Ansprüche, wobei das Trockenpulver eine Medikamentenzusammensetzung mit mindestens einem Wirkstoff für einen bestimmten Träger enthält.

## Revendications

1. Un assemblage de doseur (10) à utiliser avec un inhalateur à poudre sèche activé par la respiration (12), comprenant :
un embout (14) qui définit un passage (16) pour la libération de la poudre sèche et fournit de l'air au port d'alimentation (112) en poudre sèche d'une chambre (114) d'un inhalateur à poudre sèche activé par la respiration (12); et
un réservoir (18) comportant plusieurs cartouches (20) qui contiennent les doses pré mesurées de poudre sèche, des doses de poudre sèche étant contenues dans les cartouches du réservoir, l'un des réservoirs (18) et l'embout (14) mobiles par rapport à l'autre réservoir (18) et embout (14) pour un positionnement séquentiel des cartouches (20) dans le passage de libération (16) de l'embout (14) et un joint (46) pour assurer l'étanchéité de chacune des cartouches (20) du réservoir (18) de manière hermétique à l'air avant le positionnement de la cartouche (20) dans le passage de libération (16) de l'embout (14). Ce joint (46) comprend un film fixé au réservoir et recouvrant les cartouches (20) de manière étanche à l'air;
où une basse pression activée par la respiration dans la chambre (114) de l'inhalateur (12) provoque un débit d'air au travers du passage de libération (16) de la poudre sèche, le faisant passer dans le port d'alimentation (122) de la poudre sèche. Le débit d'air entraîne la poudre sèche de la cartouche de poudre sèche (20) située dans le passage (16) dans la chambre pour que le patient utilisant l'inhalateur (12) puisse l'inhaler;
où une basse pression activée par la respiration dans la chambre (114) de l'inhalateur (12) provoque un débit d'air au travers du passage de libération (16) de la poudre sèche, le faisant passer dans le port d'alimentation (122) de la poudre sèche. Le débit d'air entraîne la poudre sèche de la cartouche de poudre sèche (20) située dans le passage (16) dans la chambre pour que le patient utilisant l'inhalateur (12) puisse l'inhaler;
il se distingue également par le fait que l'embout comprend aussi un élément (48) pour percer le film (46) au-dessus de chaque cartouche (20) avant que la cartouche (20) soit positionnée dans le passage de libération (16) de l'embout (14).

2. Un assemblage conforme à la revendication 1, où le réservoir (18) est mobile par rapport à l'embout (14) pour un positionnement séquentiel des multiples cartouches (20) de poudre sèche dans le passage de libération (16) de l'embout (14).

3. Un assemblage conforme à la revendication 2, où le réservoir (18) est annulaire de sorte que la rotation du réservoir annulaire (18) positionne séquentiellement la pluralité des cartouches de poudre sèche (20) dans le passage de libération (16) de l'embout (14).

4. Un assemblage conforme à l'une des revendications 1 à 3, qui comprend aussi un élément pour indiquer le nombre de cartouches de poudre sèche contenant de la poudre sèche.

5. Un assemblage conforme à la revendication 4, où l'embout (14) couvre la pluralité des cartouches de poudre sèche (20) du réservoir (18) et l'élément d'indication comprend une partie transparente (70) de l'embout (14).

6. Un assemblage conforme à l'une des revendications 1 à 5, où l'un des réservoirs (18) et embout (14) sont mobiles par rapport à l'autre réservoir et embout grâce à plusieurs incréments discrets, où à chaque incrément, l'une des multiples cartouches de poudre sèche (20) est positionnée dans le passage de libération (16) de l'embout (14).

7. Un assemblage conforme à l'une des revendications 1 à 6, où l'un des réservoirs (18) et embout (14) sont mobiles par rapport à l'autre réservoir et embout dans un seul sens.

8. Un assemblage conforme à l'une des revendications 1 à 7, adapté de sorte que le réservoir (18) et l'embout (14) ne soient pas mobiles l'un par rapport à l'autre après que toutes les cartouches de poudre sèche (20) du réservoir (18) ont été positionnées dans le passage de libération (16) de poudre sèche du boîtier (142).

9. Un assemblage conforme à l'une des revendications 1 à 8, où:
l'un des réservoirs (18) et embout (14) comprend plusieurs dents (50) ; et
l'autre réservoir (18) et embout (14) comprend un cliquet flexible (52) qui passe de façon séquentielle sur les dents (50) pendant le mouvement de l'un des réservoirs (18) et embout (14) par rapport à l'autre réservoir (18) et embout (14), où le réservoir (18) est dans l'un des nombreux incréments discrets lorsque le cliquet (52) est entre les dents (50).

10. Un assemblage conforme à la revendication 9, où chacune des nombreuses dents (50) possède une face en biseau (54) permettant le passage du cliquet (52) dans un sens et une face droite (56) empêchant le passage du cliquet dans l'autre sens.

11. Un assemblage conforme à la revendication 9, où les nombreuses dents (50) comprennent une dent (58) dont les faces sont toutes les deux droites pour empêcher le passage du cliquet (52) au-delà de ladite dent (58).

12. Un assemblage conforme à l'une des revendications précédentes, où le film (46) fixé au réservoir (18) et couvrant la poudre sèche dans les cartouches de manière étanche à l'air comprend un film en plastique.

13. Un assemblage conforme a l'une des revendications 1 à 12, où le passage de libération (16) de la poudre sèche de l'embout comprend un diffuseur (22).

14. Un assemblage conforme à l'une des revendications 1 à 13 où :
le réservoir (18) est annulaire de sorte que la rotation de l'un des réservoirs (18) et embout (14) par rapport a l'autre réservoir et embout positionne séquentiellement les nombreuses cartouches de poudre sèche (20) dans le passage de libération (16) de l'embout (14), le réservoir (18) possédant une surface supérieure (28) qui délimite les cartouches (20) ; et
l'embout (14) comporte une surface inférieure (36) placée au-dessus de la surface supérieure (28) du réservoir, la surface inférieure (36) définissant le passage de libération (16) de la poudre sèche qui passe radialement vers l'intérieur depuis une autre partie jusqu'à une partie interne de l'embout (14).

15. Un assemblage conforme à l'une des revendications 1 à 14, qui comprend aussi des éléments (66, 68) pour fixer l'embout (14) au réservoir (18).

16. Un assemblage conforme à l'une des revendications 1 à 15, qui comprend aussi des éléments (74, 148) pour fixer l'assemblage (10) à une chambre (110) d'un inhalateur (12).

17. Un inhalateur à poudre sèche activé par la respiration (12) comprenant un assemblage (10) conforme à l'une des revendications 1 à 16, ainsi que :
une chambre (114) placée le long d'un axe (A) entre une première extrémité (118) et une deuxième (120) ;
un port d'alimentation en poudre sèche (122) à la première extrémité (118) de la chambre (114) en communication fluide avec le port d'alimentation (16) en poudre sèche de l'embout (14) ; et
un port de sortie (132) à la deuxième extrémité de la chambre (120);
où une basse pression activée par la respiration au port de sortie (132) produit un débit d'air dans la chambre (114) au travers du port d'alimentation en poudre sèche (122), avec le débit d'air au travers du port d'alimentation en poudre sèche (122) qui entraine la poudre sèche dans la chambre (120) depuis la cartouche (20) du réservoir (18) placé dans le passage de libération (16).

18. Un inhalateur conforme à la revendication 17, qui comprend aussi un dé-agglomérateur (110) qui comporte :
une paroi interne (112) qui définit la chambre (114) placée le long d'un axe (A) depuis la première extrémité (118) à la deuxième (120) ;
au moins un port d'entrée (124) dans la paroi interne (112) à côte de la première extrémité (118) de la chambre (114) permettant une communication fluide entre une zone externe au dé-agglomérateur et la première extrémité (118) de la chambre (114) ;
où une basse pression activée par la respiration au port de sortie (132) produit aussi un débit d'air dans la chambre (114) au travers du port d'entrée (124, 125).

19. Un inhalateur conforme à la revendication 18, où le dé-agglomérateur (110) comprend aussi des palettes (126) à la première extrémité (118) de la chambre (114) placées au moins en partie radialement vers l'extérieur depuis l'axe (A) de la chambre (114), chacune des palettes (126) possédant une surface oblique (128) dirigée au moins en partie dans un sens transversal à l'axe (A).

20. Un inhalateur conforme à l'une des revendications 18 et 19, où la chambre (114) du dé-agglomérateur (110) comprend des sections disposées de manière transversale par rapport à l'axe (A), les sections décroissant depuis la première extrémité (118) jusqu'à la deuxième extrémité (120) de la chambre (114).

21. Un inhalateur conforme à l'une des revendications 18 à 20, où :
le port d'alimentation en poudre sèche (112) du dé-agglomérateur (110) est dirigé dans un sens totalement parallèle à l'axe (A) ;
le port de sortie (132) est positionné de manière totalement transversale à l'axe (A) ; et
au moins un des ports d'entrée (124, 125) est placé dans un sens totalement transversal à l'axe (A) et parfaitement tangentiel par rapport à la chambre (114).

22. Un inhalateur conforme à la revendication 21, où au moins l'un des ports d'entrée (124, 125) du dé-agglomérateur (110) comprend deux ports d'entrée diamétralement opposés (124 et 125).

23. Un inhalateur conforme à l'une des revendications 18 à 22, où :
le dé-agglomérateur (110) comprend une base semblable à une coupelle (140) fermée par un boîtier (142) pour former la chambre (114), la base (140) constituant la paroi intérieure (112) et la deuxième extrémité (120) de la chambre (114) ainsi que le port de sortie (132), le boîtier (142) constituant la première extrémité (118) de la chambre (114), les palettes (126) et le port d'alimentation (122) ; la base (140) et le boîtier (142) constituant ensemble au moins l'un des ports d'entrée (124, 125), le boîtier (142) comprenant un guide cylindrique (144) qui s'étend vers le haut de la chambre et une évacuation (146) qui s'étend vers le haut dans le guide cylindrique depuis le port d'alimentation (122) ;
le réservoir (18) est annulaire et possède une surface circonférentielle intérieure (24) qui s'appuie coaxialement sur le guide cylindrique (144) du dé-agglomérateur (110) pour permettre au réservoir (18) de tourner par rapport au dé-agglomérateur (110), le réservoir (18) possédant une surface supérieure (28) qui définit les cartouches de poudre sèche (20) ; et
l'embout (14) comprend une surface inférieure (36) placée par-dessus la surface supérieure (28) du réservoir (18), la surface inférieure (34) constituant le passage de libération (16) de la poudre sèche, qui s'étend radialement vers l'intérieur depuis une partie extérieure vers une partie intérieure de l'embout (14), l'embout (14) comprenant aussi un capot (44) qui s'étend vers le bas depuis la partie intérieure et placé par-dessus l'évacuation (146) du dé-agglomérateur (110), le capot (44) raccordant le passage de libération (16) à l'évacuation (146) et empêchant la rotation de l'embout (14) par rapport au dé-agglomérateur (110).

24. Un inhalateur conforme à l'une des revendications 18 à 23, qui comprend aussi :
des indicateurs (82) fixés au réservoir (18), correspondant aux cartouches de poudre sèche (20) ; et
un détecteur (84) monté sur le dé-agglomérateur (110) produisant un signal de dose sur un indicateur (82) passant devant le détecteur (84) au fur et à mesure du déplacement du réservoir (18) par rapport au dé-agglomérateur (110), pour positionner l'une des cartouches (20) dans le passage de libération (16) de la poudre sèche, où un signal de dose provenant du détecteur (84) indique la libération d'une seule cartouche de poudre sèche au travers de l'inhalateur (12).

25. Un inhalateur conforme à la revendication 24, qui comprend aussi :
un compteur (non illustré) qui indique le total du nombre de signaux de dose rapportés par le détecteur (84) ;
une horloge qui donne l'heure de chaque signal de dose rapporté par le détecteur (84) ;
un processeur (80) qui propose des calculs prédéfinis en fonction du total donné par le compteur et des heures indiquées par l'horloge ;
une mémoire pour enregistrer les calculs issus des 5 processeurs (80) ; et
un élément (86) pour transmettre les calculs enregistrés à un dispositif à distance afin d'exploiter ces calculs.

26. Un assemblage ou un inhalateur conforme à l'une quelconque des revendications précédentes, où chaque cartouche (20) du réservoir (18) contient une seule dose de poudre sèche.

27. Un assemblage ou un inhalateur conforme à l'une quelconque des revendications prédécentes, où la poudre sèche comprend une composition médicamenteuse dont au moins l'un des agents actifs adhère à un vecteur de particules.
